# EUROPÄISCHE PATENTSCHRIFT

(11) **EP 1 523 275 B1**
(45) Veröffentlichungstag und Bekanntmachung des Hinweises auf die Patenterteilung: **16.11.2005**
(21) Anmeldenummer: 03763816.0
(22) Anmeldetag: 11.07.2003
(51) Int. Cl.: A61B 17/04, A61B 17/28, B25B 7/08

(54) **MEDIZINISCHES GREIF- UND/ODER HALTEINSTRUMENT**
MEDICAL GRIPPING AND/OR RETAINING INSTRUMENT
INSTRUMENT MEDICAL DE PRISE ET/OU DE MAINTIEN

(30) Priorität: 16.07.2002 DE 10232061
(43) Veröffentlichungstag der Anmeldung: 20.04.2005
(73) Patentinhaber: Karl Storz GmbH & Co. KG, 78532 Tuttlingen (DE)
(72) Erfinder: FRANK, Tim, Wormit, Fife KY16 9TY (GB)
(74) Vertreter: Hofmeister, Frank
(86) Internationale Anmeldenummer: PCT/EP2003/007525
(87) Internationale Veröffentlichungsnummer: WO 2004/006779

(56) Entgegenhaltungen:
- DE-A- 4 412 171
- DE-C- 32 617
- GB-A- 477 199
- GB-A- 805 066
- US-A- 2 679 779
- US-A- 5 944 718
- US-B1- 6 386 076

## Beschreibung

Die Erfindung betrifft ein medizinisches Greif- und/oder Halteinstrument, insbesondere Nadelhalter, mit einer aus zwei Handgriffen bestehenden Handhabe und einem aus zwei Maulteilen bestehenden und über die Handhabe betätigbaren Halteteil, wobei die Maulteile über eine Schwenkachse relativ zueinander verschwenkbar sind.

Medizinische Greif- und/oder Halteinstrumente werden für die verschiedensten Einsatzzwecke, beispielsweise als Zangen oder Nadelhalter verwendet. Diese bekannten Greif- und/oder Halteinstrumente sind meist zangenförmig aufgebaut und weisen zwei um eine gemeinsame Schwenkachse verschwenkbare Hebelarme auf, deren distale Enden die Maulteile des Halteteils bilden, wohingegen deren proximale Enden als Handgriffe der Handhabe dienen. Aufgrund der Lagerung der verschwenkbaren Maulteile um eine gemeinsame Schwenkachse, ist zwischen den sich öffnenden bzw. sich schließenden Maulteilen ein Öffnungswinkel ausgebildet, um den zueinander versetzt die inneren Greifflächen der Maulteile im Bezug zur Instrumentenlängsachse an der Schwenkachse gelagert sind. Dieser maulartige Öffnungswinkel bleibt bestehen, wenn in der Haltestellung ein Gegenstand klemmend zwischen den Greifflächen der Maulteile angeordnet ist.

Wenn der zu haltende Gegenstand einen starren im wesentlichen kreiszylindrischen Schaft aufweist, wie dies bei einer von einem Nadelhalter zu haltenden Nadel der Fall ist, kann der Gegenstand nur dann verdrehsicher zwischen den Maulteilen gehalten werden, wenn der zu haltende Gegenstand in einem rechten Winkel zur Instrumentenlängsachse angeordnet ist, da nur diese Winkellage einen linienförmigen Kontakt zwischen einer Greiffläche eines Maulteils und dem Gegenstand ermöglicht.

Sobald der Gegenstand nicht rechtwinklig zur Instrumentenlängsachse zwischen den Maulteilen angeordnet ist, bewirkt der zwischen den Maulteilen ausgebildete Öffnungswinkel, dass die sich schließenden Maulteile den Gegenstand ausschließlich mit den am nächsten zur Schwenkachse angeordneten Bereichen der Greifflächen klemmend erfassen, so dass nur ein punktförmiger Kontakt zwischen den Greifflächen und dem Gegenstand möglich ist. Diese nur punkförmige Lagerung des Gegenstandes zwischen den Maulteilen ist sehr instabil, was dazu führt, dass ein solchermaßen gehaltener Gegenstand sich bei der geringsten Belastung relativ zur Instrumentenlängsachse verdreht. Ein solches Verdrehen ist für eine von einem Nadelhalter ergriffene und geführte Nadel nicht tolerierbar.

Aus der US-5 944 718-A ist ein elektro-chirurgisches Instrument mit einer aus zwei Handgriffen bestehenden Handhabe und einem aus zwei Maulteilen bestehenden und über die Handhabe betätigbaren Halteteil bekannt, wobei das bewegliche Maulteil über eine Betätigungsstange um 180° um die Längsachse des Maulteils verdrehbar ist, damit die Greiffläche des beweglichen Maulteils ein zwischen den Maulteilen gelagertes Objekt klemmend oder schneidend kontaktieren kann. Zwar ermöglicht diese bekannte Konstruktion die Umstellung eines Maulteils zur Variation der Anlage an dem zu ergreifenden Objekt, jedoch ermöglicht diese Konstruktion mit der 180° Verstellung nicht das sichere Ergreifen beispielsweise konischer Gegenstände.

Davon ausgehend liegt der Erfindung die **Aufgabe** zugrunde, ein medizinisches Greif- und/oder Halteinstrument der eingangs genannten Art so auszugestalten, dass ein von den Maulteilen ergriffener Gegenstand unabhängig von dessen Winkellage bezüglich der Instrumentenlängsachse verdrehsicher von den Maulteilen gehalten wird.

Die **Lösung** dieser Aufgabenstellung ist erfindungsgemäß dadurch gekennzeichnet, dass das eine Maulteil zur Aufnahme der Schwenkachse zwei parallele, voneinander beabstandete Seitenwände aufweist und das andere Maulteil im Bereich zwischen den beiden parallelen Seitenwänden als Steg ausgebildet ist, der mit Abstand zu den beiden Seitenwänden derart auf der gemeinsamen Schwenkachse gelagert ist, dass das Maulteil des Halteteils um die Längsachse des Maulteils verdrehbar ist.

Aufgrund der verdrehbeweglichen Lagerung des mindestens einen Maulteils ist es mit dem erfindungsgemäß ausgestalteten Greif- und/oder Halteinstrument erstmalig möglich, einen von den Maulteilen ergriffenen Gegenstand in beliebiger Winkellage positionsfest und sicher zu halten, da es dank der Verdrehbeweglichkeit möglich ist, den Öffnungswinkel der Maulteile in Querrichtung der Maulteile variabel zu gestalten.

Zur Erzielung der Kippbeweglichkeit des einen Maulteils gegenüber dem anderen Maulteil wird vorgeschlagen, dass der die Nabe bildende Steg im Bereich der Lagerung auf der Schwenkachse derart ausgebildet ist, dass die der Nabe entsprechende Bohrung im Steg sich von beiden Seiten zur Mitte der Bohrung hin bis auf den Außendurchmesser der Schwenkachse verjüngt, so dass dieser vorzugsweise linienförig auf einer umlaufenden Kreisbahn auf der Schwenkachse gelagert ist.

Die verdrehsichere Lagerung des von den Maulteilen zu haltenden Gegenstands wird vorteilhafterweise dadurch erzielt, dass die Greifflächen der Maulteile an einen zwischen den Maulteilen des Halteteils gehaltenen Gegenstand, unabhängig von dessen Winkellage bezüglich der Instrumentenlängsachse, so anliegen, dass diese Greifflächen den Gegenstand jeweils an mindestens zwei voneinander beabstandeten Kontaktpunkten kontaktieren. Dieses Ergreifen des Gegenstands über jeweils zwei Kontaktpunkte je Maulteil erhöht den Reibwiderstand und verhindert die Ausbildung eines Drehpunktes, wie dies bei nur einem punktförmigen Kontakt der Fall ist.

Gemäß einer bevorzugten Ausführungsform der Erfindung kontaktiert mindestens eine Greiffläche eines Maulteils den Gegenstand im wesentlichen linienförmig. Der linienförmige Kontakt stellt dabei die zuverlässigste Lagerung des Gegenstandes dar, wenn dieser kreiszylindrisch ausgebildet ist.

Um auch Gegenstände sicher und fest zwischen den Maulteilen halten zu können, die keinen gleichbleibenden Außendurchmesser aufweisen, wird erfindungsgemäß vorgeschlagen, dass die Greiffläche eines Maulteils eben und die Greiffläche eines anderen, mit diesem Maulteil zusammenwirkenden Maulteils in Längsrichtung dieses Maulteils gesehen von der Kontaktfläche mit dem zu haltenden Gegenstand fort gewölbt ausgebildet ist, wodurch das Maulteil mit der gewölbten Greiffläche nur mit seinen Außenkanten am zu haltenden Gegenstand angreift und zwei verdrehsichere, voneinander beabstandete Kontaktpunkte erzeugt.

Gemäß einer praktischen Ausführungsform der Erfindung wird vorgeschlagen, dass ein Handgriff der Handhabe einstückig starr mit einem Maulteil des Halteteils ausgebildet ist, während der andere Handgriff der Handhabe um einen Gelenkpunkt verschwenkbar mit dem anderen Maulteil des Halteteils verbunden ist, und dass die Handgriffe über mindestens ein Führungselement, insbesondere ein Federelement, miteinander verbunden sind, wobei das Führungselement mit einem Ende an einem Lagerpunkt im Bereich des proximalen Endes des einen Handgriffes und mit dem anderen Ende an einem Lagerpunkt im mittleren Bereich des anderen Handgriffes gelagert ist.

Um zu ermöglichen, dass trotz der verdrehbewegliche Lagerung des Maulteils der mit diesem Maulteil verbundene Handgriff der Handhabe ohne seitliche Kippbewegung zum Öffnen und Schließen der Maulteile auf und ab bewegt werden kann, wird weiterhin vorgeschlagen, dass der verschwenkbare Handgriff oder das verschwenkbare Maulteil relativ zum jeweils anderen Bauteil gesehen kippbeweglich auf der Gelenkachse des Gelenkpunktes gelagert ist. Diese kippbewegliche Lagerung zwischen Handgriff und Maulteil ermöglicht zusammen mit dem zwischen den Handgriffen angeordneten Führungselement eine stabile und verkippsichere Bewegung des Handgriffs der Handhabe, da die Lagerung im Bereich des Gelenkpunktes die mögliche Verdrehung des Maulteils wieder ausgleicht.

Weiterhin wird mit der Erfindung vorgeschlagen, dass bei einem erfindungsgemäßen Instrument, bei dem ein Teil eines Maulteils des Halteteils gegenüber mindestens einem anderen, mit diesem Maulteil zusammenwirkenden Maulteil ausschließlich um die Längsachse des Maulteils verdrehbar gelagert ist, in dem Steg des einen Maulteils ein senkrecht zur Instrumentenlängsachse verlaufender Stift festgelegt ist, der in den Seitenwänden des anderen Maulteils gelagert ist. Dieser Stift, der vorzugsweise um die Instrumentenlängsachse kippbar in den Seitenwänden gelagert ist, soll eine Relativbewegung des kippbaren Maulteils in Längsrichtung des anderen Maulteils verhindern.

Weitere Merkmale und Vorteile der Erfindung ergeben sich anhand der nachfolgenden Beschreibung der zugehörigen Zeichnung, in der ein Ausführungsbeispiel eines erfindungsgemäßen medizinischen Greif- und/oder Halteinstruments nur beispielhaft dargestellt ist In der Zeichnung zeigt:
- Fig. 1: eine schematische Seitenansicht einer ersten Ausführungsform eines erfindungsgemäßen medizinischen Greif- und/oder Halteinstruments;
- Fig. 2a: einen Schnitt entlang der Schnittlinie II-II, die Maulteile in gerader Stellung darstellend;
- Fig. 2b: einen Schnitt entlang der Schnittlinie II-II, die Maulteite in gekippter Stellung darstellend;
- Fig. 3a: einen Schnitt entlang der Schnittlinie III-III, die Maulteile in gerader Stellung darstellend;
- Fig. 3b: einen Schnitt entlang der Schnittlinie III-III, die Maulteile in gekippter Stellung darstellend;
- Fig. 4a: eine schematische Draufsicht auf ein Halteteil eines-erfindungsgemäßen Greif- und/oder Halteinstruments mit einer rechtwinklig zur Instrumentenachse zwischen den Maulteilen gehaltenen Nadel;
- Fig. 4b: eine schematische Seitenansicht der Darstellung gemäß Fig. 4a;
- Fig. 4c: einen schematischen Querschnitt entlang der Linie IVc-IVc gemäß Fig. 4a;
- Fig. 5a: eine schematische Draufsicht auf ein Halteteil eines erfindungsgemäßen Greif- und/oder Halteinstruments mit einer nicht rechtwinklig zur Instrumentenachse zwischen den Maulteilen gehaltenen Nadel;
- Fig. 5b: eine schematische Seitenansicht der Darstellung gemäß Fig. 5a und
- Fig. 5c: einen schematischen Querschnitt entlang der Linie Vc-Vc gemäß Fig. 5a.

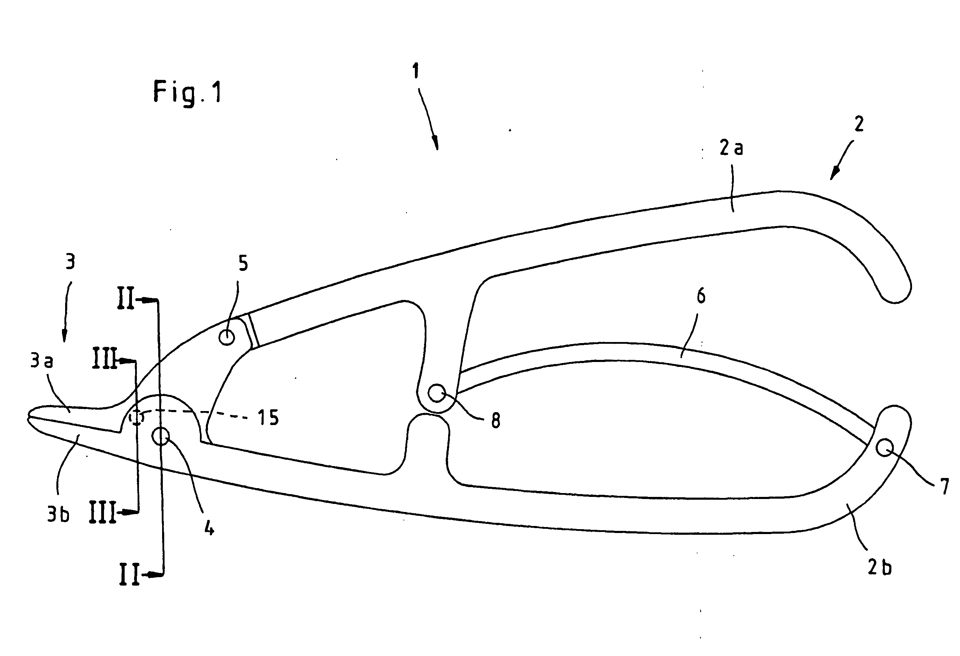

Bei dem in Abbildung Fig. 1 dargestellten medizinischen Greif- und/oder Halteinstrument handelt es sich um einen chirurgischen Nadelhalter 1.

Der prinzipiell zangenartig aufgebaute Nadelhalter 1 besteht im wesentlichen aus einer proximalseitigen Handhabe 2 mit zwei Handgriffen 2a und 2b sowie einem distalseitigen Halteteil 3 mit zwei Maulteilen 3a und 3b, die um eine Schwenkachse 4 relativ zueinander verschwenkbar sind.

Bei der in Abbildungen Fig. 1 dargestellten ersten Ausführungsform des Nadelhalters 1 sind der Handgriff 2b sowie das Maulteil 3b einstückig starr miteinander verbunden, wohingegen der andere Handgriff 2a sowie das andere Maulteil 3a über einen Gelenkpunkt 5 verschwenkbar miteinander verbunden sind. Bei dieser dargestellten Ausführungsform kreuzen sich die Maulteile 3a, 3b bzw. die Handgriffe 2a, 2b nicht.

Wie weiter aus der Abbildung Fig. 1 ersichtlich, ist zwischen den Handgriffen 2a und 2b der Handhabe 2 ein als Blattfeder 6 ausgebildetes Führungselement angeordnet, welches mit einem Ende an einem Lagerpunkt 7 im Bereich des proximalen Endes des starren Handgriffes 2b und mit dem anderen Ende an einem Lagerpunkt 8 im mittleren Bereich des verschwenkbaren Handgriffes 2a gelagert ist. Die Blattfeder 6 dient dazu, die Handgriffe 2a, 2b bzw. die Maulteile 3a, 3b des Halteteils 3 in definierten Endstellungen zu fixieren, nämlich in einer offenen Endstellung des Halteteils 3 und in einer geschlossenen Endstellung des Halteteils 3.

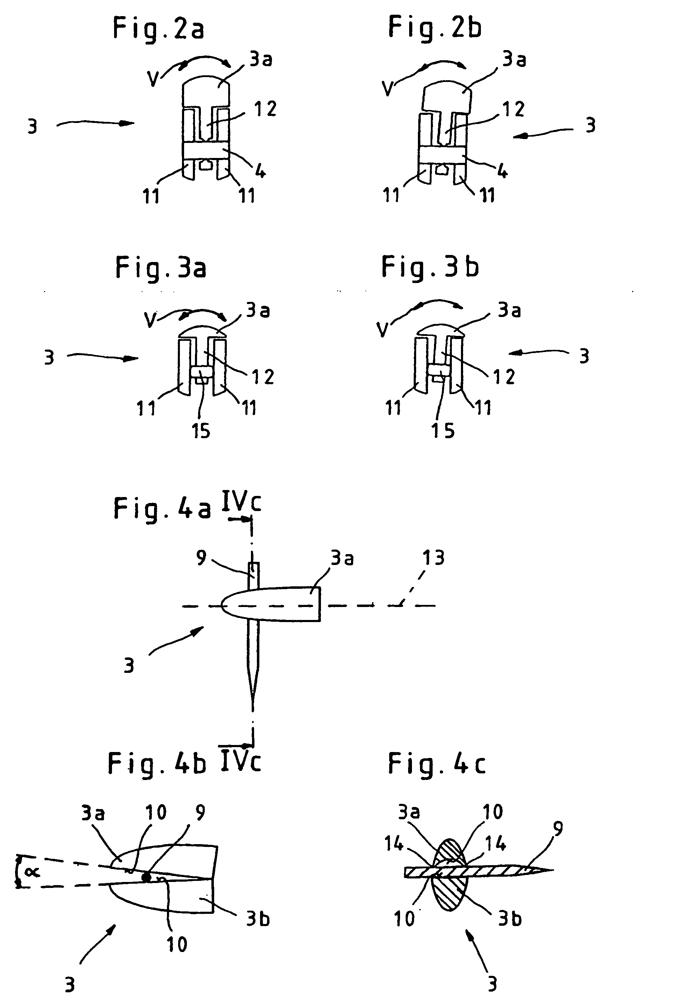

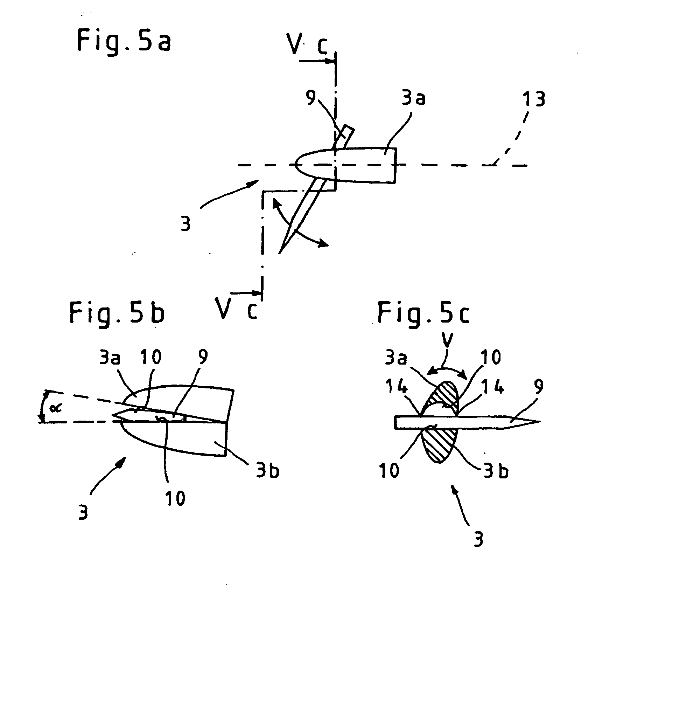

Das Halteteil 3 des Nadelhalters 1 kontaktiert eine zwischen den Maulteilen 3a, 3b zu haltende Nadel 9 über Greifflächen 10, wie dies den Abbildungen Fig. 4b, 4c und 5b, 5c zu entnehmen ist. Bei der dargestellten Ausführungsform ist die Greiffläche 10 des unteren Maulteils 3b eben und die Greiffläche 10 des oberen Maulteils 3a in Längsrichtung des Maulteils 3a nach innen gewölbt ausgebildet.

Wie aus den Schnittdarstellungen gemäß Fig. 2a bis 3b ersichtlich, ist das obere Maulteil 3a um die Längsachse dieses Maulteils 3a gegenüber dem unteren Maulteil 3b verdrehbar gelagert. Diese verdrehbewegliche Lagerung in Richtung des Pfeiles V wird bei der dargestellten Ausführungsform dadurch erzielt, dass das untere Maulteil 3b zur Aufnahme der Schwenkachse 4 zwei parallele, voneinander beabstandete Seitenwände 11 aufweist und das obere Maulteil 3a über einen Steg 12 zwischen den beiden parallelen Seitenwänden 11 und mit Abstand zu den beiden Seitenwänden 11 auf der Schwenkachse 4 gelagert ist, wobei der die Nabe der Lagerung bildende Steg 12 im Bereich der Lagerung auf der Schwenkachse 4 beidseitig eingeschnürt ausgebildet ist, wie dies die Abbildungen Fig. 2a und 2b verdeutlichen.

Aufgrund dieser Einschnürung am Steg 12 ergibt sich eine im wesentlichen linienförmige Lagerung des Stegs 12 auf einer umlaufenden Kreisbahn auf der Schwenkachse 4, so dass das obere Maulteil 3a wegen des seitlichen Abstandes zwischen dem Steg 12 und den Seitenwänden 11 des unteren Maulteils 3b, in Längsrichtung des Maulteils 3a betrachtet, nach rechts oder nach links verdrehbar ist.

Wie sich aus einem Vergleich der Darstellungen in Fig. 4c und 5c ergibt, ist diese verdrehbewegliche Lagerung des oberen Maulteils 3a notwendig, wenn die zwischen den Maulteilen 3a, 3b angeordnete Nadel 9 auch dann verdrehsicher und fest gehalten werden soll, wenn diese nicht rechtwinklig zur Instrumentenlängsachse 13 zwischen den Greifflächen 10 der Maulteile 3a, 3b angeordnet ist.

Bei der Darstellung gemäß Fig. 4a bis 4c ist die Nadel 9 rechtwinklig zur Instrumentenlängsachse 13 zwischen den Maulteilen 3a und 3b angeordnet. Aufgrund des kreiszylindrischen Schaftes der Nadel 9 mit einem im wesentlichen gleichbleibenden Durchmesser liegt die Nadel 9 bei der rechtwinkligen Ausrichtung vollflächig auf der ebenen Greiffläche 10 des unteren Maulteils 3b auf und wird von zwei Kontaktpunkten 14 der Greiffläche 10 des oberen Maulteils 3a kontaktiert. Dieser linienförmige Kontakt am unteren Maulteil 3b zusammen mit der Zweipunktlagerung am oberen Maulteil 3a bewirkt eine verdrehsichere Lagerung der Nadel 9 im Halteteil 3.

Da sich der Abstand zwischen den Maulteilen 3a, 3b des Halteteils 3 bei jedem gewählten Öffnungswinkel α mit wachsender Entfernung von der Schwenkachse 4 vergrößert, ist es nicht möglich, eine nicht rechtwinklig zur Instrumentenlängsachse 13 angeordnete Nadel 9 verdrehsicher über die Greifflächen 10 der Maulteile 3a, 3b zu ergreifen, wenn die Maulteile 3a, 3b ausschließlich um die Schwenkachse 4 verschwenkbar, ansonsten aber starr gelagert sind.

Sobald die Nadel 9 nicht rechtwinklig zur Instrumentenlängsachse 13 zwischen den Maulteilen 3a, 3b angeordnet ist, bewirkt der zwischen den Maulteilen 3a, 3b ausgebildete Öffnungswinkel α, dass die sich schließenden Maulteile 3a, 3b die Nadel 9 ausschließlich mit den am nächsten zur Schwenkachse 4 angeordneten Bereichen der Greifflächen 10 klemmend erfassen, so dass nur ein punktförmiger Kontakt zwischen den Greifflächen 10 und der Nadel 9 möglich ist. Diese nur punktförmige Lagerung der Nadel 9 zwischen den Maulteilen 3a, 3b ist aber sehr instabil, was dazu führt, dass eine solchermaßen gehaltene Nadel 9 sich bei der geringsten Belastung relativ zur Instrumentenlängsachse 13 verdrehen kann.

Die verdrehbewegliche Lagerung des oberen Maulteils 3a erlaubt es der Greiffläche 10 des oberen Maulteils 3a aber, sich durch Verdrehen an die unterschiedlichen Abstände zwischen den Maulteilen 3a, 3b anzupassen und die Nadel 9 wiederum so zu ergreifen, dass das obere Maulteil 3a an zwei voneinander beabstandeten Kontaktpunkten 14 an der Nadel 9 angreift und diese zur Ausbildung eines im wesentlichen linienförmigen Kontakts auf die Greiffläche 10 des unteren Maulteils 3b drückt, wie dies der Abbildung Fig. 5c zu entnehmen ist. Dieser linienförmige Kontakt am unteren Maulteil 3b zusammen mit der Zweipunktlagerung am oberen Maulteil 3a bewirkt wiederum eine verdrehsichere Lagerung der Nadel 9 im Halteteil 3.

Wie die Schnittdarstellungen gemäß Fig. 3a und 3b weiterhin zeigen, weist der Steg 12 des oberen Maulteils 3a einen in dem Steg 12 festgelegten, senkrecht zur Instrumentenlängsachse 13 verlaufenden Stift 15 auf, der in den Seitenwänden 11 des unteren Maulteils 3b gelagert ist und an seinen Enden vorzugsweise gerundet ausgebildet ist. Dieser um die Instrumentenlängsachse 13 kippbar in den Seitenwänden 11 gelagerte Stift 15 soll eine Relativbewegung des oberen Maulteils 3a in Richtung der Instrumentenlängsachse 13 zum unteren Maulteil 3b verhindern.

Um zu verhindern, dass das Verdrehen des Maulteils 3a gleichzeitig auch eine Verdrehung des mit diesem Maulteil 3a verbundenen Handgriffs 2a bewirkt, ist es möglich, die Lagerung des Maulteils 3a am Handgriff 2a im Bereich der Gelenkachse des Gelenkpunktes 5 ähnlich der Lagerung auf der Schwenkachse 4 so auszugestalten, dass die Lagerung ein Verkippen der Bauteile 2a, 3a relativ zueinander ermöglicht. Die diese Verkippung ermöglichende Nabe kann dabei entweder am Maulteil 3a oder am Handgriff 2a ausgebildet sein.

Diese kippbewegliche Lagerung zwischen Handgriff 2a und Maulteil 3a ermöglicht zusammen mit dem zwischen den Handgriffen angeordneten, als Blattfeder 6 ausgebildeten Führungselement eine stabile und verkippsichere Bewegung des Handgriffs 2a der Handhabe 2, da diese Lagerung im Bereich des Gelenkpunktes 5 die mögliche Verdrehung des Maulteils 3a wieder ausgleicht.

### Bezugszeichenliste

- 1: Nadelhalter
- 2: Handhabe
- 2a: Handgriff
- 2b: Handgriff
- 3: Halteteil
- 3a: Maulteil
- 3b: Maulteil
- 3c: starrer Teil
- 3d: verdrehbarer Teil
- 4: Schwenkachse
- 5: Gelenkpunkt
- 6: Blattfeder
- 7: Lagerpunkt
- 8: Lagerpunkt
- 9: Nadel
- 10: Greiffläche
- 11: Seitenwand
- 12: Steg
- 13: Instrumentenlängsachse
- 14: Kontaktpunkt
- 15: Stift

- V: Kippbeweglichkeit
- α: Öffnungswinkel

## Patentansprüche

1. Medizinisches Greif- und/oder Halteinstrument, insbesondere Nadelhalter, mit einer aus zwei Handgriffen (2a, 2b) bestehenden Handhabe (2) und einem aus zwei Maulteilen (3a, 3b) bestehenden und über die Handhabe (2) betätigbaren Halteteil (3), wobei die Maulteile (3a, 3b) über eine Schwenkachse (4) relativ zueinander verschwenkbar sind,
**dadurch gekennzeichnet,**
**dass** das eine Maulteil (3b) zur Aufnahme der Schwenkachse (4) zwei parallele, voneinander beabstandete Seitenwände (11) aufweist und das andere Maulteil (3a) im Bereich zwischen den beiden parallelen Seitenwänden (11) als Steg (12) ausgebildet ist, der mit Abstand zu den beiden Seitenwänden (11) derart auf der gemeinsamen Schwenkachse (4) gelagert ist, dass das Maulteil (3a) des Halteteils (3) um seine Längsachse verdrehbar ist.

2. Medizinisches Greif- und/oder Halteinstrument nach Anspruch 1, **dadurch gekennzeichnet, dass** der die Nabe bildende Steg (12) im Bereich der Lagerung auf der Schwenkachse (4) derart ausgebildet ist, dass die der Nabe entsprechende Bohrung im Steg (12) sich von beiden Seiten zur Mitte der Bohrung hin bis auf den Außendurchmesser der Schwenkachse (4) verjüngt.

3. Medizinisches Greif- und/oder Halteinstrument nach Anspruch 1 oder 2, **dadurch gekennzeichnet, dass** der Steg (12) im wesentlichen linienförmig auf der Schwenkachse (4) gelagert ist.

4. Medizinisches Greif- und/oder Halteinstrument nach mindestens einem der Ansprüche 1 bis 3, **dadurch gekennzeichnet, dass** Greifflächen (10) der Maulteile (3a, 3b) an einem zwischen den Maulteilen (3a, 3b) des Halteteils (3) gehaltenen Gegenstand, unabhängig von dessen Winkellage bezüglich einer Instrumentenlängsachse (13), so anliegen, dass die Greifflächen (10) den Gegenstand jeweils an mindestens zwei voneinander beabstandeten Kontaktpunkten (14) kontaktieren.

5. Medizinisches Greif- und/oder Halteinstrument nach Anspruch 4, **dadurch gekennzeichnet, dass** mindestens eine Greiffläche (10) eines Maulteils (3a oder 3b) den Gegenstand im wesentlichen linienförmig kontaktiert.

6. Medizinisches Greif- und/oder Halteinstrument nach Anspruch 4 oder 5, **dadurch gekennzeichnet, dass** die Greiffläche (10) eines Maulteils (3b) eben und die Greiffläche (10) eines anderen, mit diesem Maulteil (3b) zusammenwirkenden Maulteils (3a) in Längsrichtung dieses Maulteils (3a) gesehen von der Kontaktfläche mit dem zu haltenden Gegenstand fort gewölbt ausgebildet ist.

7. Medizinisches Greif und/oder Halteinstrument nach mindestens einem der Ansprüche 1 bis 6, **dadurch gekennzeichnet, dass** ein Handgriff (2b) der Handhabe (2) einstückig starr mit einem Maulteil (3b) des Halteteils (3) ausgebildet ist, während der andere Handgriff (2a) der Handhabe (2) um einen Gelenkpunkt (5) verschwenkbar mit dem anderen Maulteil (3a) des Halteteils (3) verbunden ist, und dass die Handgriffe (2a, 2b) über mindestens ein Führungselement, insbesondere eine Blattfeder (6), miteinander verbunden sind, wobei das Führungselement mit einem Ende an einem Lagerpunkt (7) im Bereich des proximalen Endes des einen Handgriffes (2b) und mit dem anderen Ende an einem Lagerpunkt (8) im mittleren Bereich des anderen Handgriffes (2a) gelagert ist.

8. Medizinisches Greif- und/oder Halteinstrument nach Anspruch 7, **dadurch gekennzeichnet, dass** der verschwenkbare Handgriff (2a) oder das verschwenkbare Maulteil (3a) relativ zum jeweils anderen Bauteil (3a oder 2a) gesehen kippbeweglich auf der Gelenkachse des Gelenkpunktes (5) gelagert ist.

9. Medizinisches Greif- und/oder Halteinstrument nach mindestens einem der Ansprüche 1 bis 8, **gekennzeichnet durch** einen in dem Steg (12) des einen Maulteils (3a) festgelegten, senkrecht zur Instrumentenlängsachse (13) verlaufenden Stift (15), der in den Seitenwänden (11) des anderen Maulteils (3b) gelagert ist.

10. Medizinisches Greif- und/oder Halteinstrument nach Anspruch 9, **dadurch gekennzeichnet, dass** der Stift (15) um die Instrumentenlängsachse (13) kippbar in den Seitenwänden (11) gelagert ist.

## Claims

1. Medical gripping and/or retaining instrument, in particular a needle holder, with a handle (2) made up of two handgrips (2a, 2b), and with a retaining part (3) which is made up of two jaw parts (3a, 3b) and can be operated via the handle (2), the jaw parts (3a, 3b) being able to pivot relative to one another via a pivot shaft (4), **characterized in that** one jaw part (3b), for receiving the pivot shaft (4), has two parallel side walls (11) arranged at a distance from one another, and the other jaw part (3a) is designed, in the area between the two parallel side walls (11), as a web (12) which, at a distance from both side walls (11), is mounted on the common pivot shaft (4) in such a way that the jaw part (3a) of the retaining part (3) can turn about its longitudinal axis.

2. Medical gripping and/or retaining instrument according to Claim 1, **characterized in that** the web (12) forming the hub, in the area where it is mounted on the pivot shaft (4), is designed in such a way that the bore in the web (12) corresponding to the hub narrows, from both sides towards the centre of the bore, to the external diameter of the pivot shaft (4).

3. Medical gripping and/or retaining instrument according to Claim 1 or 2, **characterized in that** the web (12) is mounted substantially linearly on the pivot shaft (4).

4. Medical gripping and/or retaining instrument according to at least one of Claims 1 to 3, **characterized in that** gripping surfaces (10) of the jaw parts (3a, 3b) bear on an object held between the jaw parts (3a, 3b) of the retaining part (3), irrespective of the angle position of said object relative to a longitudinal axis (13) of the instrument, in such a way that the gripping surfaces (10) each make contact with the object at at least two contact points (14) spaced apart from one another.

5. Medical gripping and/or retaining instrument according to Claim 4, **characterized in that** at least one gripping surface (10) of a jaw part (3a or 3b) makes contact with the object in an essentially linear manner.

6. Medical gripping and/or retaining instrument according to Claim 4 or 5, **characterized in that** the gripping surface (10) of one jaw part (3b) is flat, and the gripping surface (10) of another jaw part (3a) interacting with this jaw part (3b) is designed curving away from the contact surface with the object to be retained, when viewed in the longitudinal direction of this jaw part (3a).

7. Medical gripping and/or retaining instrument according to at least one of Claims 1 to 6, **characterized in that** one handgrip (2b) of the handle (2) is made rigidly integral with a jaw part (3b) of the retaining part (3), while the other handgrip (2a) of the handle (2) is connected to the other jaw part (3a) of the retaining part (3) in such a way as to be pivotable about a hinge point (5), and **in that** the handgrips (2a, 2b) are connected to one another via at least one guide element, in particular a leaf spring (6), the guide element being mounted with one end on a bearing point (7) in the area of the proximal end of one handgrip (2b) and being mounted with its other end on a bearing point (8) in the central area of the other handgrip (2a).

8. Medical gripping and/or retaining instrument according to Claim 7, **characterized in that** the pivotable handgrip (2a) or the pivotable jaw part (3a) is mounted on the hinge axis of the hinge point (5) so as to be able to tilt relative to the respective other structure (3a or 2a).

9. Medical gripping and/or retaining instrument according to at least one of Claims 1 to 8, **characterized by** a pin (15) which is secured in the web (12) of one jaw part (3a), extends perpendicular to the longitudinal axis (13) of the instrument and is mounted in the side walls (11) of the other jaw part (3b).

10. Medical gripping and/or retaining instrument according to Claim 9, **characterized in that** the pin (15) is mounted in the side walls (11) so that it can tilt about the longitudinal axis (13) of the instrument.

## Revendications

1. Instrument médical de préhension et/ou de retenue, en particulier porte-aiguille, comportant une manette (2) constituée par deux poignées (2a, 2b) et un élément de retenue (3) constitué par deux parties de mâchoire (3a, 3b) et susceptible d'être actionné par la manette (2), les parties de mâchoire (3a, 3b) étant mobiles en pivotement l'une par rapport à l'autre via un axe de pivotement (4),
**caractérisé en ce que**
pour recevoir l'axe de pivotement (4) l'une des parties de mâchoire (3b) comprend deux parois latérales parallèles (11) écartées l'une de l'autre, et l'autre partie de mâchoire (3a) est réalisée sous forme de barrette (12) dans la zone entre les deux parois latérales parallèles (11), barrette qui est montée à distance des deux parois latérales (11) sur l'axe de pivotement commun (4) de telle sorte que la partie de mâchoire (3a) de l'élément de retenue (3) est capable de tourner autour de son axe longitudinal.

2. Instrument médical de préhension et/ou de retenue selon la revendication 1, **caractérisé en ce que** la barrette (12) formant le moyeu est réalisée dans la zone du montage sur l'axe de pivotement (4) de telle sorte que le perçage correspondant au moyeu et ménagé dans la barrette (12) va en se rétrécissant depuis les deux côtés vers le milieu du perçage jusqu'à rejoindre le diamètre extérieur de l'axe de pivotement (4).

3. Instrument médical de préhension et/ou de retenue selon l'une ou l'autre des revendications 1 et 2, **caractérisé en ce que** la barrette (12) est montée sensiblement en forme linéaire sur l'axe de pivotement (4).

4. Instrument médical de préhension et/ou de retenue selon l'une au moins des revendications 1 à 3, **caractérisé en ce que** des surfaces de préhension (10) des parties de mâchoire (3a, 3b) s'appuient contre un objet retenu entre les parties de mâchoire (3a, 3b) de l'élément de retenue (3), et ceci indépendamment de sa position angulaire par rapport à un axe longitudinal (13) de l'instrument, de telle sorte que les surfaces de préhension (10) viennent en contact avec l'objet à au moins deux points de contact respectifs (14) écartés l'un de l'autre.

5. Instrument médical de préhension et/ou de retenue selon la revendication 4, **caractérisé en ce qu'**au moins une surface de préhension (10) d'une partie de mâchoire (3a ou 3b) vient en contact sensiblement linéaire avec l'objet.

6. Instrument médical de préhension et/ou de retenue selon l'une ou l'autre des revendications 4 et 5, **caractérisé en ce que** la surface de préhension (10) d'une partie de mâchoire (3b) est réalisée de façon plane et la surface de préhension (10) d'une autre partie de mâchoire (3a) coopérant avec cette partie de mâchoire (3b) est réalisée de façon bombée, vue en direction longitudinale de cette partie de mâchoire (3a), en éloignement de la surface de contact avec l'objet à retenir.

7. Instrument médical de préhension et/ou de retenue selon l'une au moins des revendications 1 à 6, **caractérisé en ce qu'**une poignée à main (2b) de la manette (2) est réalisée d'un seul tenant rigidement avec une partie de mâchoire (3b) de l'élément de retenue (3), tandis que l'autre poignée à main (2a) de la manette (2) est reliée à l'autre partie de mâchoire (3a) de l'élément de retenue (3) en étant mobile en pivotement autour d'un point d'articulation (5), et **en ce que** les poignées à main (2a, 2b) sont reliées l'une à l'autre via au moins un élément de guidage, en particulier un ressort à lame (6), l'élément de guidage étant monté par une extrémité à un point de montage (7) dans la zone de l'extrémité proximale de l'une des poignées à main (2b) et étant monté par l'autre extrémité à un point de montage (8) dans la zone médiane de l'autre poignée à main (2a).

8. Instrument médical de préhension et/ou de retenue selon la revendication 7, **caractérisé en ce que** la poignée à main (2a) mobile en pivotement ou la partie de mâchoire (3a) mobile en pivotement est montée sur l'axe d'articulation du point d'articulation (5) en étant mobile en basculement, vue par rapport à l'autre composant respectif (3a ou 2a).

9. Instrument médical de préhension et/ou de retenue selon l'une au moins des revendications 1 à 8, **caractérisé par** une goupille (15) immobilisée dans la barrette (12) de l'une des parties de mâchoire (3 a) et s'étendant perpendiculairement à l'axe longitudinal (13) de l'instrument, goupille qui est montée dans les parois latérales (11) de l'autre partie de mâchoire (3b).

10. Instrument médical de préhension et/ou de retenue selon la revendication 9, **caractérisé en ce que** la goupille (15) est montée dans les parois latérales (11) en étant mobile en basculement autour de l'axe longitudinal (13) de l'instrument.
